(19) Europäisches Patentamt
European Patent Office
Office européen des brevets

(11) EP 4 086 914 A1

(12) EUROPEAN PATENT APPLICATION

(43) Date of publication:
09.11.2022 Bulletin 2022/45

(21) Application number: 22171736.6

(22) Date of filing: 05.05.2022

(51) International Patent Classification (IPC):
G16H 20/17 (2018.01)    G16H 50/70 (2018.01)
A61M 5/172 (2006.01)    A61B 5/145 (2006.01)

(52) Cooperative Patent Classification (CPC):
G16H 20/17; G16H 50/70; A61B 5/14532;
A61M 5/1723

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 05.05.2021 US 202163184251 P

(71) Applicant: Insulet Corporation
Acton MA 01720 (US)

(72) Inventors:
• NARAYANASWAMI, Rangarajan
  Weston (US)
• ZHENG, Yibin
  Hartland (US)
• JANTZ, Jay
  Acton (US)
• LEE, Joon Bok
  Acton (US)

(74) Representative: Peterreins Schley
Patent- und Rechtsanwälte PartG mbB
Hermann-Sack-Straße 3
80331 München (DE)

(54) USING MACRONUTRIENT INFORMATION TO OPTIMIZE INSULIN DOSING

(57) The disclosed embodiments are directed to systems and methods for providing optimized, individualized bolus dosing of insulin based on a macronutrient profile of meals ingested by the patient. Optimized bolus dosing may be provided by varying the overall quantity of insulin delivered in the post-prandial window, as well as the split between a portion of the insulin delivered immediately after the meal and a portion of the insulin delivered later in the post-prandial window, based on an analysis of the macronutrient profile of the meal and the behavior of the blood glucose trace of the patient from past meals.

Automatic Insulin Delivery (AID) System
100

FIG. 1

**Description**

**BACKGROUND**

Insulin delivery for meal compensation is a critical component in blood glucose control in automatic insulin delivery (AID) systems for people with diabetes. Insulin dosages calculated for meal compensation are commonly based on the number of grams of carbohydrates in the meal. The recommended bolus insulin dosage is typically calculated by dividing the number of grams of carbohydrates in the meal by the patient's insulin to carbohydrate ratio.

Factors other than the number of grams of carbohydrates in a meal effect the size of the bolus dose and the timing of the delivery of the bolus dose of insulin required to offset the post-prandial absorption of glucose into the bloodstream. For example, the overall macronutrient profile of a meal, including the ratios of fat and protein to carbohydrates, and the quantities of fat, protein and carbohydrates in the meal will affect not only the amount of glucose which enters the bloodstream, but also the rate and timing of the absorption of the glucose from the meal into the bloodstream. Ideally, a bolus dose of insulin is delivered to the user that matches the timing of the absorption of the glucose into the bloodstream.

As an example, high fat meals tend to cause a delay in gastric emptying and require more insulin than low-fat meals for the same number of grams of carbohydrates. Differences between low fat, low protein (LF-LP) vs. high fat high protein (HF-HP) meals can be summarized as such:

> (1) The peak time for blood glucose absorption following a HF-HP meal is significantly delayed by approximately 30 min compared to a LF-LP meal, for an identical insulin dose; and (2) The total area under the blood glucose curve (amount of blood glucose absorbed versus time) for the HF-HP meal is almost twice that for the LF-LP meal. There is a significant difference in blood glucose values, which tend to remain significantly elevated for the same insulin dose from 180 minutes onwards of meal ingestion for the HF-HP meal. For a low fat, high protein (LF-HP) meal, blood glucose values tend to be elevated from 210 minutes onwards compared to a LF-LP meal. For a high fat, low protein (HF-LP) meal, the blood glucose values matched the (LF-HP) values from 180 minutes onwards. However, the HF-LP meal had a lower blood glucose excursion in the first 60-90 minutes compared to all other meals.

In these various scenarios, fat and protein have an additive effect on the delayed post-prandial rise of blood glucose levels. Interestingly, protein tends to have a protective effect on the development of hypoglycemia after meals. Conversely, a HF-LP meal can increase chances of hypoglycemia after meals because of delayed gastric emptying. The causes of late sustained hyperglycemia when the meals are HF-HP are not well understood. Possible explanations point to proteins in meals promoting gluconeogenesis and increased glucagon secretion. Fats in meals impair insulin sensitivity, enhance hepatic glucose production, delay gastric emptying, and increase the peak time and amplitude of the glucose response. Studies have shown that to achieve target glucose levels following a HF-HP meal, as much as 65% more insulin was required with a split of 30%/70% over 2.4 hours.

As can be seen, there is significant variability in the blood glucose response after meals with varying proportions of fat and protein. The optimum split for the bolus dose for an individual may vary from patient to patient. Optimal delivery patterns may range from a 10%/90% split to a 50%/50% split. The optimized dose may range from an additional 65% of the insulin calculated using the patient's insulin-to-carbohydrate ratio with some requiring as much as 75% additional insulin.

These combined factors point to the desirability of having an individualized approach to insulin delivery based on information regarding the overall macronutrient profile of ingested meals and the reaction of each individual to various types of meals. The inclusion of fat and protein in the calculation of the optimal bolus dose of insulin for meal compensation would therefore lead to an improvement in the blood glucose control for both open loop and hybrid closed loop systems. As such, it would be desirable to provide a system and method to determine the optimal bolus dose and bolus split for individual patients, based on the macronutrient profile of ingested meals and the patient's past history of controlling blood glucose levels following the various categories of meals.

**DEFINITIONS**

As used herein, the term "***insulin***" should be interpreted to include bolus doses of insulin, GLP-1, pramlintide, or co-formulations of GLP-1, pramlintide, and/or insulin.

As used herein, the term "***bolus split***" refers to splitting a bolus dose of insulin into a first portion, to be delivered at the beginning of the post-prandial window, and a second portion, to be delivered at a later time or over an extended period of time starting at some point after the first portion is delivered.

As used herein, the terms **"patient"** and "user" are used interchangeably and are meant to include persons suffering from type 1 or type 2 diabetes mellitus who are using the disclosed invention to control bolus doing of insulin.

As used herein, an "***open-loop system***" refers to a drug delivery system that delivers basal and bolus insluin based on input from a user, and not in an automated fashion.

As used herein, a "***closed-loop system***" refers to a drug delivery system that delivers a basal dose at a varying rate in response to data received from a CGM, in an automated fashion.

As used herein, a "*hybrid closed-loop system*" refers to a drug delivery system that delivers basal insulin at a varying rate in response to data received from a CGM, in an automated fashion, but delivers bolus insulin in a manual fashion, based on input from a user.

## SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

Disclosed herein are systems and methods for providing optimized, individualized bolus dosing of insulin based on macronutrient information of meals ingested by the patient.

In a first aspect of the invention, a machine-learning model that is trained for each individual patient is provided that predicts the post-prandial blood glucose trace of the patient based on the macronutrient profile of the ingested meal and given a specific proposed bolus dose of insulin.

In another aspect of the invention, an improved meal input interface for the patient is provided which allows the specification of the overall macronutrient profile of an ingested meal to be provided.

In another aspect of the invention, an adaptive insulin delivery strategy for meal compensation is provided in which both the bolus dose quantity and the bolus split ratio are optimized for the patient.

In another aspect of the invention, an individualized machine-learning model is provided that can determine the macronutrient profile of a meal based on an actual post-prandial blood glucose trace.

In another aspect of the invention, a method for determining the timing of the delivery of the delayed portion of the bolus split in a plurality of doses during the post-prandial window is provided.

In a final aspect of the invention, feedback to the patient in the form of meal catalogs and blood glucose response to various meals is provided.

In preferred embodiments of the invention, the recommended bolus dose may be provided to an AID system which may include a wearable drug delivery device providing closed loop or hybrid closed loop basal and bolus dosing of the patient.

A primary embodiment of the invention may comprise any combination of the aspects discussed above, while other embodiments of the invention may contain subsets of the various aspects.

## BRIEF DESCRIPTION OF THE DRAWINGS

To easily identify the discussion of any particular element or act, the most significant digit or digits in a reference number refer to the figure number in which that element is first introduced.

**FIG. 1** is a block diagram of an AID system including the bolus (and basal) dosing application of the present invention.

**FIG. 2** is a block diagram showing the training and use of the machine-learning model for providing the post-prandial blood glucose trace prediction.

**FIGS. 3A-3C** show several embodiments of user interfaces enabling the user to enter the macronutrient profile of an ingested meal.

**FIG. 4** is a block diagram of a process of the system for adjusting the recommended bolus dose and bolus split to obtain desired post-perennial blood glucose metrics.

**FIG. 5** is a flowchart showing the process by which the recommended bolus dose and bolus split is determined.

**FIG. 6** is a flowchart showing one exemplary embodiment of an algorithm for adjusting the bolus dose and bolus split based on the post-prandial blood glucose metrics.

**FIG. 7** is a flowchart showing the selection of an initial bolus dose and bolus split used for generating the post-prandial blood glucose prediction.

**FIG. 8** is a block diagram of a method for determining the macronutrient profile of a meal based on a post-prandial blood glucose trace.

## DETAILED DESCRIPTION

Systems and methods in accordance with the present disclosure will now be described more fully with reference to the accompanying drawings, where one or more embodiments or various aspects of the invention are shown. The systems and methods may be embodied in many different forms and are not to be construed as being limited to the embodiments set forth herein. Instead, these embodiments are provided so the disclosure will be thorough and complete, and will fully convey the scope of the systems and methods to those skilled in the art. Each of the systems and methods disclosed herein provides one or more advantages over conventional systems and methods.

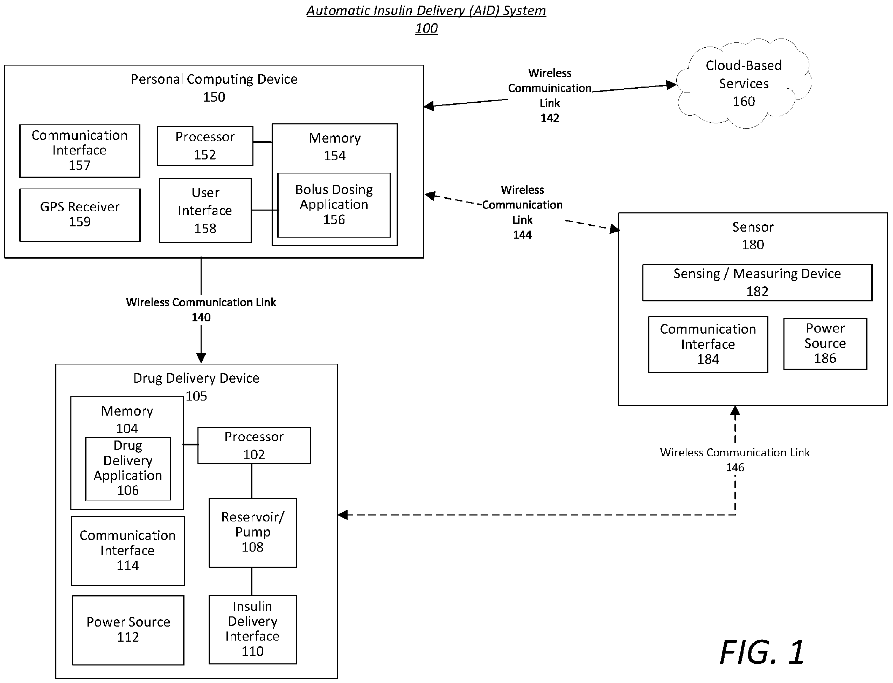

FIG. 1 is a block diagram showing an AID system which includes a bolus dosing application 156 implementing the various novel aspects of the invention described in the Summary above and in more detail below. In a primary embodiment of the invention, the bolus dosing application 156 may be implemented as a software application executing on a personal computing device 150. Personal computing device 150 may be configured with a processor 152 and a memory 154 containing the bolus dosing application 156. Personal computing device 150 may be further configured with a user interface 158 which may be used by the bolus dosing application 156 to enable interaction with a user. The bolus dosing application 156 may be configured to accept meal information input via user interface 158. The bolus dosing application 156 is configured to receive post-prandial blood glucose traces of the user. In some embodiments, the blood glucose traces may be received from a sensor 180, for example, a continuous blood glucose monitor (CGM), directly via communication link 144 with sensor 180, or indirectly via drug delivery device 105 via wireless communication link 140, which communicates with sensor 180 via wireless connection 146. Alternatively, the user may enter the blood glucose levels directly via user interface 158. The bolus dosing application 156 may further communicate with cloud-based services 160 via communication link 142 as described below. In alternate embodiments of the invention, the bolus dosing application 156 may be implemented as a cloud-based service 160 and accessed via wireless communication link 142 with the user's personal computing device 150.

In some embodiments, personal computing device 150 may comprise, for example, a smartphone, a tablet device, a smartwatch, or any other personal mobile computing device capable of running bolus dosing application 156 and communicating with the drug delivery device 105, cloud-based services 160 and sensor 180 via any well-known wireless communication protocol.

The bolus dosing application 156 may track and correlate the macronutrient profiles of meals eaten by the user with blood glucose traces of the user for a predetermined period of time (hereinafter referred to as the "post-prandial window") after the user has ingested the meal, and may inform a drug delivery algorithm 106 executing on drug delivery device 105 such as to enable the drug delivery algorithm 106 to adjust the bolus dose and bolus split of insulin delivered to the user to more effectively control the blood glucose levels of the user during the post-prandial window.

In one aspect of the invention, an individualized post-prandial blood glucose prediction model 216 (hereinafter, "model 216") is developed for each user. The model 216 is trained on various input data to provide a post-prandial blood glucose prediction 220 which may be, for example, a prediction of the blood glucose levels of the user at various intervals during the post-prandial window. For example, in a preferred embodiment of the invention, the model 216 could predict the post-prandial blood glucose levels of the user at intervals of 30 minutes during a post-prandial window lasting 3 to 4 hours after ingestion of the meal. In other embodiments, other shorter or longer intervals may be used and the overall length of the post-prandial window may vary without departing from the scope of the invention.

In one embodiment of the invention, a new user may be provided with a model 216 which has been trained for a large population of users or for a cluster of users having similar characteristics to the new user. Bolus doses of insulin could be calculated for the new user and the model 216 would then be refined for the new user over a period of time based on the accuracy of the post-prandial blood glucose prediction 220, given the calculated bolus dose and feedback from a continuous glucose monitor (CGM).

In other embodiments of the invention, a new user could be provided with an un-trained model 216. In this embodiment, the bolus dose of insulin could be, during an initial training period of the model 216 (e.g., two weeks, 40 meals, etc.), based solely on the carbohydrate content of the meal ingested by the user and the user's particular insulin-to-carbohydrate ratio. After data has been collected and fed back to the model 216 during the limited training period, the model 216 could start to be used to refine the bolus dose of insulin for particular meals for the individual user.

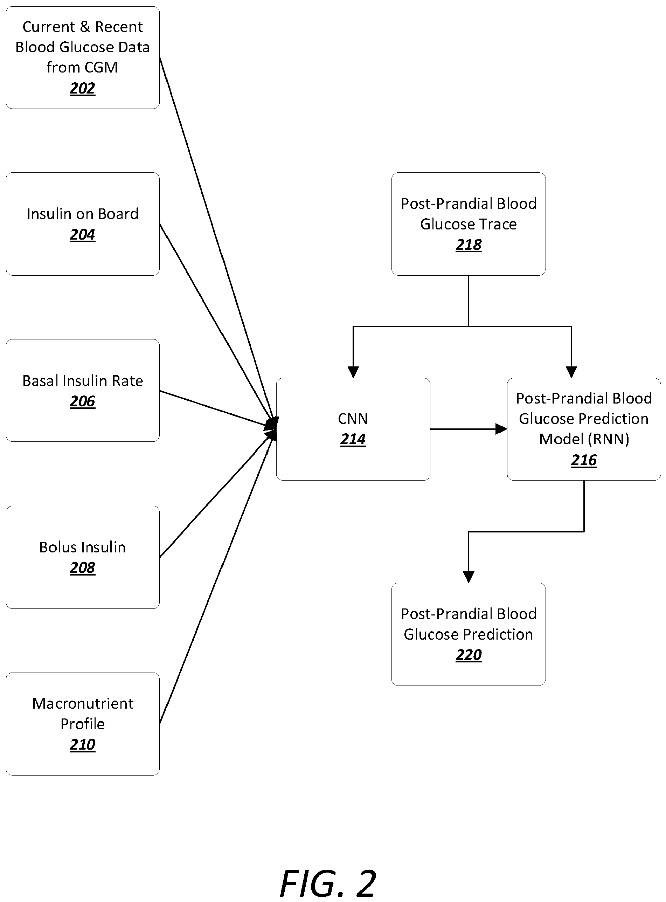

FIG. 2 is a block diagram showing the training and use of model 216. In preferred embodiments of the invention, the model may be trained on the data shown in FIG. 2 as reference numbers 202-210. In other embodiments of the invention, other data may be used as training data for the model 216. In preferred embodiments of the invention, the model may comprise a convolutional neural network 214 and a post-prandial blood glucose prediction model 216, which may be, for example, a recurrent neural network. Convolution neural network 214 may output a dilated convolution of the input data 202-210 in the form of features extracted for the input data that can be used by model 216 to generate the post-prandial blood glucose prediction 220. In other embodiments of the invention, other implementations and architectures of model 216 may be used. As an example, a generative adversarial network could be a possible candidate for implementation of model 216.

The current and recent blood glucose data from a CGM 202 may be provided as an input to model 216. Preferably, the user is provided with a CGM that provides a blood glucose trace showing the user's blood glucose level at predetermined intervals, (e.g., every five minutes, two minutes, one minute, or the like) during the post-prandial window of a predetermined length (e.g. 4 hours) after ingestion of a meal.

The insulin on board 204, the current basal rate 206 and the bolus insulin 208 may also be provided as an input to CNN 214. The insulin on board 204 is a measure of the total available insulin in the body of the user. Once injected into the body, insulin remains active for a period of time, referred to as Duration of Insulin action. Typically, this period of time is on the order of approximately three to four hours but can extend for up to 6 hours. As such, the insulin on board is the insulin present in the body of the user because of past insulin delivery. The basal rate 206 is the current basal insulin delivery rate. If the AID system is an open-loop system, a constant or user-input basal rate 206 is assumed. However, if the AID system is a closed-loop or hybrid closed-loop system in which the current readings from the CGM are used to adjust basal delivery rate, the closed-loop (or hybrid closed-loop) basal delivery rate is used. The bolus insulin 208 is the insulin delivered for the current meal. Given the current insulin on board 204, the basal insulin rate 206, and the bolus insulin dose for the current meal 208, the insulin present in the user's body at any post-prandial time can be computed.

The macronutrient profile 210 of the ingested meal is also used as input to the CNN 214. Preferably, the macronutrient profile 210 comprises the total quantity of carbohydrates, fat and protein from a meal that the user has recently ingested or is about to ingest. The total quantity, in most instances, is entered by the user using a user interface designed to allow the user to easily enter this information. In certain embodiments, the user interface used to enter the macronutrient profile of the meal is provided by or part of bolus dosing application 156 running on a personal computing device 150. In other embodiments, the user interface may be provided by another application running on the user's personal computing device 150.

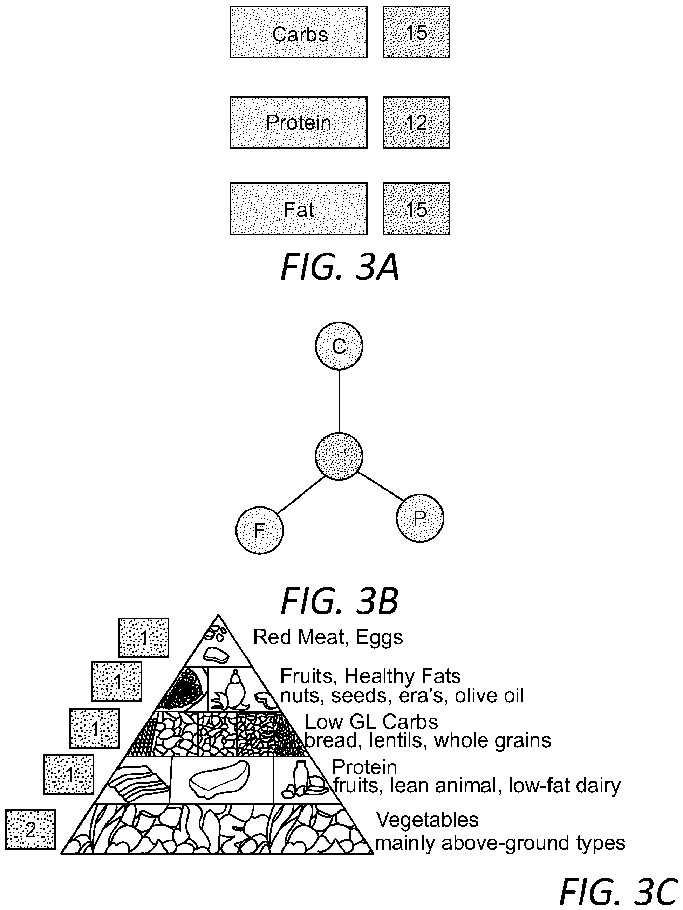

Several examples of user interfaces are shown in **FIGS. 3A-3C.** In the exemplary user interface of **FIG. 3A**, the user is able to enter the actual quantity of carbohydrates, fats, and proteins in the meal. The values can be entered utilizing a keyboard provided by the user interface 158 of personal computing device 150 or may be entered using a form of sliding input to increase or decrease the displayed value. **FIG. 3B** shows a second exemplary embodiment of a user interface in which the relative portions of carbohydrates, fats, and proteins can be used to capture the profile of the meal. In this interface the carbohydrate (C), fat (F), and protein (P) ratios may be displayed either on the center blue button, or, alternatively, the C, F and P buttons may change context and display this information. In another aspect of this embodiment of the user interface, the center blue button may be moved between the C, F and P buttons to show the relative proportions of the carbohydrates, fats and proteins in the meal. **FIG. 3C** shows yet a third exemplary embodiment of user interface in which a traditional "food pyramid" is used. This embodiment allows users to specify the food types and serving sizes. For example, the user may select different parts of the food pyramid and when selected, an input field is presented where the user may indicate the serving size. Alternatively, the user may tap the particular portion of the food pyramid repeatedly to increase the serving size of the particular food group that the user intends to consume, and the serving size may cycle from 1 to 2 to 3 and then back to 1 in case the user mistakenly repeatedly taps a particular portion of the food pyramid. Another example of a user interface allowing the user to enter the macronutrient profile of the meal is disclosed in pending U.S. Patent Application 63/184,241, the contents of which are incorporated herein in their entirety. Given the information which may be entered in any one of the exemplary user interfaces, the meal type category (i.e., LF-LP, HF-HP, LF- HP, HF-LP) as well as estimates of the actual numbers of carbohydrates, fat, and protein may be deduced.

In one embodiment, the categorization of the meal may be based on a ratio of the fat and protein grams to the number of carbohydrate grams in the meal. For example, a meal categorized as a HF or HP meal may be a meal wherein the ratio of the number of fat grams to the number of carbohydrate grams or the ratio of the number of protein grams to the number of carbohydrate grams in the meal exceeds 0.5. Likewise, a meal may be categorized as an LF or LP meal if the ratio of the number of fat grams to the number of carbohydrate grams or the ratio of the number of protein grams to the number of carbohydrate grams in the meal is below 0.2. When the ratios are between 0.2 and 0.5, the meal may be neither high or low in fat or protein. For simplicity, in one embodiment, meals having ratios higher than 0.5 may be considered high fat or high protein, while meals having ratios below 0.5 may be considered low-fat or low protein meals. As would be realized by one of skill in the art, in other embodiments of the invention, different criteria or different absolute numbers may be used to categorize the meal.

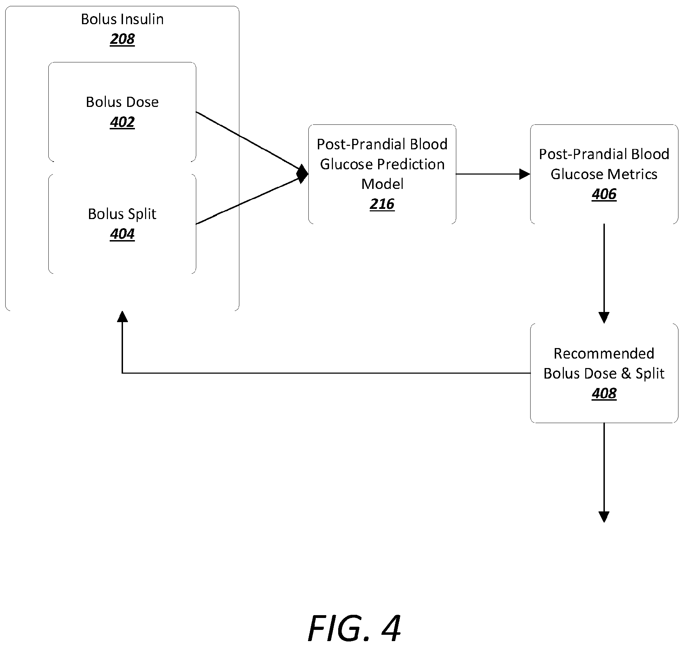

Based on the inputs 202-210 discussed above, the model 216 produces the post-prandial blood glucose prediction 220. The model 216 may then be used to determine an optimal bolus dose 402 and bolus split 404 as shown in **FIG. 4.**

The model 216 may be constantly updated by feeding back the actual post-prandial blood glucose trace from the CGM into both the CNN 214 and the model 216. The data fed back into CNN 214 and model 216 may be the actual blood glucose trace from the CGM collected during the post-prandial window or may be post-prandial blood glucose metrics 406 which have been derived from the actual post-prandial blood glucose trace and which are discussed later herein.

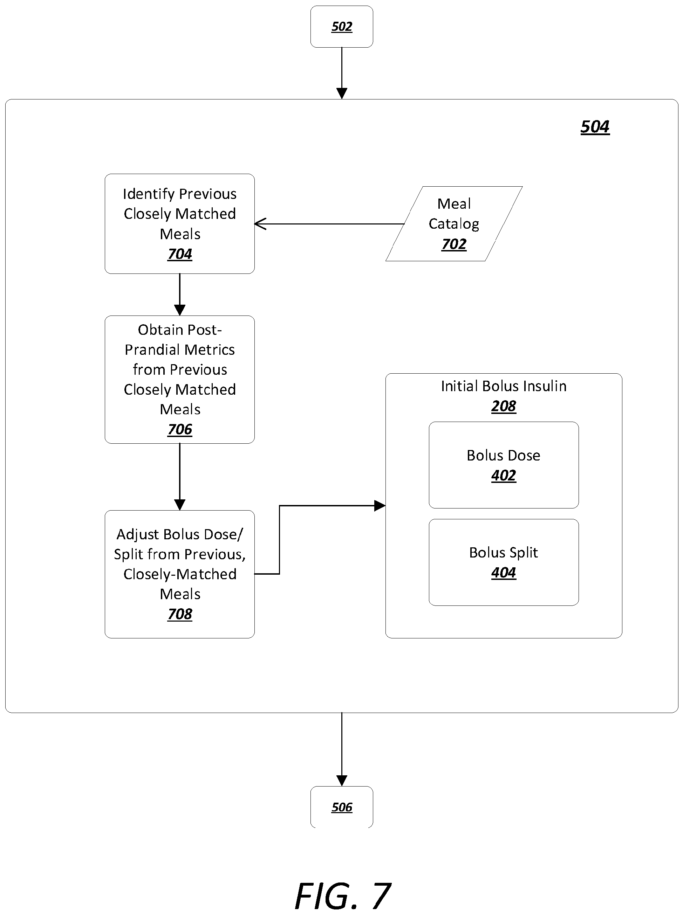

**FIG. 4** shows the process by which the model predicts an optimal bolus dose 402 and split 404, given the inputs 202-210 shown in **FIG. 2.** An initial bolus dose 402 and bolus split 404 is first determined. In earlier stages of the use of the system and method of the present invention with a new user, as previously discussed, bolus dose 402 and bolus split 404 may be based on the carbohydrate content of the meal combined with the insulin to carbohydrate ratio of the user. After the initial training period of model 216, in one embodiment, the initial bolus dose 402 and bolus split 404 may be determined by the process shown in **FIG. 7** and discussed later herein. This process looks for past, similar meals and uses the bolus dose 402 and bolus split 404 for the past, similar meals as the initial bolus dose 402 and bolus split 404 for the current meal. The bolus dose 402 and bolus split 404 may be adjusted based on various factors, including, for example, to compensate for differences in the meals. In another embodiment, the initial bolus dose 402 and bolus split 404 may be determined as a percentage of the total daily insulin for the user. As an example, the bolus dose could be assumed to be 6%, 7%, or 8% of the total daily insulin. The invention is not meant to be limited in terms of how the initial bolus dose 402 and bolus split 404 is determined, as any method may be used.

Based on the inputs 202-210, including the initial bolus dose 402 and bolus split 404, model 216 produces a prediction 220 of the post-prandial blood glucose trace as previously described. The prediction 220 of the post-prandial blood glucose trace is synthesized into a set of post-prandial blood glucose metrics 406 and the metrics 406 are used to evaluate the prediction 220. Based on the evaluation, the recommended bolus dose 402 and bolus split 404 is adjusted and used as an input for the model 216 in a next iteration. The predictive process is iterated until optimal or desired values for the post-prandial blood glucose metrics 406 are achieved and the bolus dose 402 and bolus split 404 which produced those metrics are used as the recommended dose (and timing) to be administered to the user.

The post-prandial blood glucose trace predicted by the model 216 may be synthesized into various post-prandial blood glucose metrics 406 which are used to evaluate the prediction of the model 216 and, ultimately, to provide a recommended bolus dose 402 and bolus split 404 which is used to dose the user. The post-prandial blood glucose metrics 406 may include, but are not limited to: the time to peak blood glucose level from the time of the meal ingestion, the numerical value of the peak blood glucose level, the total area under the blood glucose curve through the post-prandial window, the total area under the blood glucose curve from, for example, 180 minutes after ingestion of the meal to the end of the post-prandial window, the time that the blood glucose level was in a desired range (e.g., blood glucose values between 70 mg/dL and 180 mg/dL), the count of the hypoglycemic readings (e.g., the number of instances when the blood glucose values fell below 70 mg/dL), the count of the hyperglycemic readings (e.g., the number of instances when the blood glucose values were above 180 mg/dL), the area of the blood glucose curve over a hyperglycemic threshold (e.g., 180 mg/dL), and the area of the blood glucose curve under a hypoglycemia threshold (e.g., 70 mg/dL). In other embodiments, different metrics may be used or a subset or superset of the above-mentioned metrics 406 may be used to evaluate the prediction 220 of model 216.

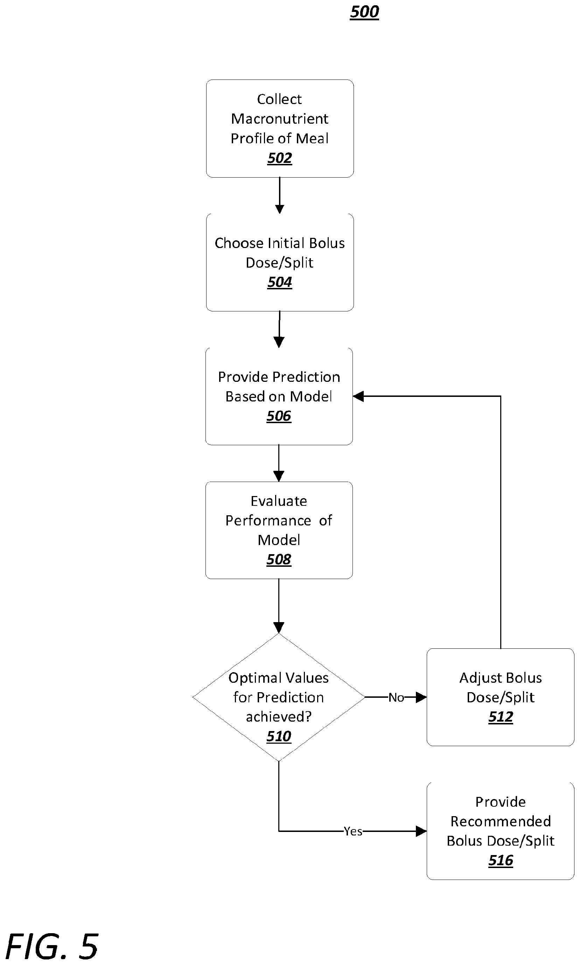

The process for adjusting the bolus dose 402 and the bolus split 404 is shown in flowchart form in **FIG. 5.** At 502, the macronutrient profile of the meal is obtained and, at 504, an initial bolus dose 402 and bolus split 404 is chosen. At 506, model 216 produces a prediction 220 for the post-prandial blood glucose trace, from which the post-prandial blood glucose metrics 406 are derived. At 508, the performance of the model 216 is evaluated using the post-prandial blood glucose metrics 406. At 510, it is determined if optimal or desired values for the post-prandial blood glucose metrics 406 have been achieved and, if not, the bolus dose 402 and bolus split 404 are adjusted at 512 and the model 216 is run again at 506 based on the new, adjusted bolus dose 402 and bolus split 404 to produce an updated post-prandial blood glucose prediction 220. If, at 510, optimal or desired values for the post-prandial blood glucose metrics 406 have been achieved, the current bolus dose 402 and bolus split 404 are used as the recommendation dose for the user.

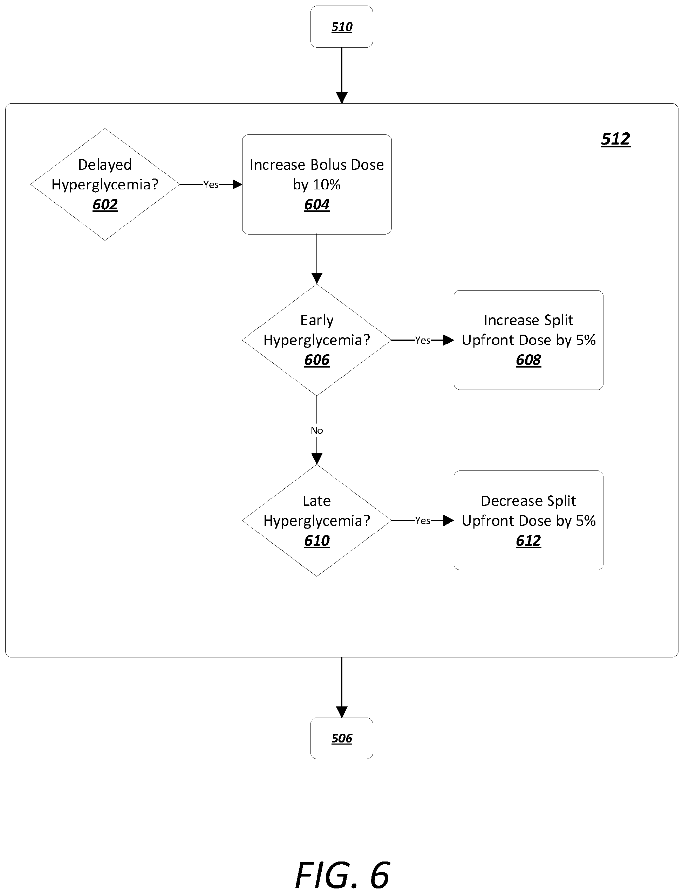

A flowchart showing an exemplary method for adjusting the bolus dose 402 and bolus split 404 in step 512 of the process is shown in **FIG. 6.** The actual algorithm for adjusting the bolus dose 402 and bolus split 404 may use the numerical values shown in **FIG. 6**, or may use different values than those shown in **FIG. 6**, or may be a completely different method than that shown in **FIG. 6**, which uses a different algorithm or criteria for determining how the bolus dose 402 and bolus split 404 should be adjusted.

The exemplary method for adjusting the bolus dose 402 and the bolus split 404 is as follows. At 602, it is determined if delayed hyperglycemia is indicated in the prediction 220 of the post-prandial blood glucose trace. If not, process 512 exits and continues at box 506 from **FIG. 5.** If delayed hyperglycemia is indicated, the bolus dose 402 may be increased by a certain percentage, for example, 10%, at block 604. At 606, it is determined if the delayed hyperglycemia occurred at an early stage of the post-prandial window, and, if so, in 608, the bolus split 404 may be adjusted. As an example, the first portion of the bolus split 404 may be increased by 5%, while the second, delayed portion of the bolus split 404 may be decreased by 5%. At 610, if the hyperglycemia occurs in a later stage of the post-prandial window, the bolus split 402 may be adjusted as shown is 612 in a different manner. For example, the first portion of the bolus split 404 may be decreased by 5% while the second, delayed portion of the bolus split 404 may be increased by 5%. As would be realized by one of skill in the art, many different combinations of percentages may be used to achieve the optimal or desired values for the post-prandial metrics 406. Additionally, the process shown in **FIG. 6** is exemplary only and not intended as limiting to the scope of the invention.

**FIG. 7** is a flowchart showing one possible embodiment of the process for determining the initial optimal values for the bolus dose 402 and bolus split 404 as previously discussed. The determination of the initial bolus dose 402 and bolus split 404 can be made more accurate by evaluating the actual post-prandial blood glucose traces from previous meals having macronutrient profiles which closely match the macronutrient profile of the current meal. Once the macronutrient profile of the current meal is obtained at 502 from **FIG. 5**, a catalog of previous meals 702 is searched and, at 704, previous meals having similar macronutrient profiles to the current meal are identified. The post-prandial metrics 406 from the identified meals are obtained in 706. Depending on specific metrics, such as the blood glucose values after 180 min (area under blood glucose curve after 180 min) the bolus dose 402 and bolus split 404 from the previous, closely-matched meal may be adjusted at 708, and used as the initial bolus dose 402 and bolus split 404 for the current meal. For example, if the blood glucose excursion after 180 min is high, the bolus split 404 from the previous, closely-matched meal may be adjusted with a higher percentage of the bolus split 404 being delayed (for example 30/70 instead of 40/60). Additionally, the bolus dose 402 from the previous, closely-matched meal may be increased by 10%. In carrying out the adaptation, the closest meals with the closest macronutrient profile to the current meal will be used.

In another aspect of the invention, meal catalog 702 is created. Meal catalog 702 contains records of all the meals for which a macronutrient profile has been entered by the user. Post-prandial blood glucose metrics 406 derived from the actual blood glucose trace associated with the meals will also be stored in meal catalog 702, as will the bolus dose 402 and bolus split 404 that produced the actual blood glucose trace. In addition, various statistics may be saved which may include, but are not limited to: the total carbs/proteins/fat consumed per day, the number of meals/snacks per day, post-prandial blood glucose profiles for the day, total insulin injected, the partition of the insulin between bolus and basal, etc. Glucose excursions from target range will be tracked. Foods which are tolerated better by the user may be identified.

A principal component analysis (PCA) may be used to assist in the building of meal catalog 702. PCA can decompose post-prandial glucose traces together with other metrics and meal parameters into independent components, where a meal will be represented by the coefficients of the independent components. The meal catalog 702 may therefore be represented by a set of coefficients.

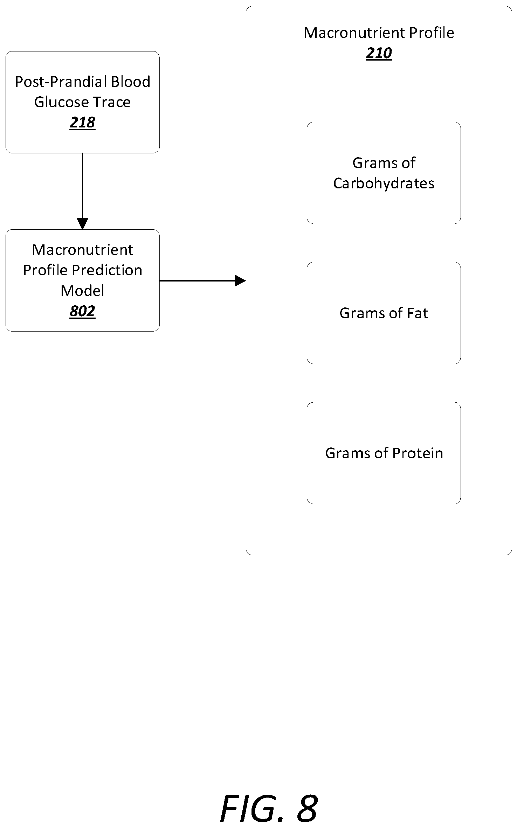

In another aspect of the invention, shown in **FIG. 8,** a macronutrient profile prediction model 802 may be provided. Macronutrient profile prediction model 802 may be, in one embodiment, a machine-learning model that is trained to predict the macronutrient profile 210 of an ingested meal based on the post-prandial blood glucose trace 218. Once several blood glucose traces from the post-prandial window are recorded in meal catalog 702, along with the associated macronutrient profiles 210 of the meals which generated the post-prandial blood glucose traces, a macronutrient profile 210 can then be generated based on an input of the post-prandial blood glucose trace 218 for the current meal. This capability can be used to generate labels for meals not having an associated macronutrient profile 210 and for adjustment of the insulin delivery by recognizing the meal type (e.g. LF-LP, HF-HP, LF-HP, HF-LF) early enough in the post-prandial window. If the meal type is recognized early enough, guidance can be provided to the insulin delivery system for adjustment. For example, if a HF-LP meal is recognized at 200 mins, the controller may increase the basal rate. If a HF-HP meal is detected early on by observing the delayed peak in the blood glucose, there is an option to increase the basal rate or add a small bolus to address the delayed hyperglycemia.

In one embodiment, the identification of the macronutrient profile 210 of the meal from the post-prandial glucose trace 218 is done by linear regression. For example, if the meal consists of two macronutrients with glucose response $m_1(t)$ and $m_2(t)$ respectively, then the proportion of each macronutrient can be determined by fitting the post prandial glucose trace $g(t)$ to a linear combination of $m_1(t)$ and $m_2(t)$ as:

$$g(t) = c_1 m_1(t) + c_2 m_2(t) + e(t)$$

where:

$c_1$, $c_2$ are carb amounts of each macronutrient; and
$e(t)$ is the residual error.

After sufficient meals (e.g. >40 or two weeks), a user's meal composition can be clustered to form meal consumption patterns. The center of each cluster represents the glucose signature of a meal composition for the user. Then, for an upcoming meal, initial glucose response can be matched to a glucose signature in the user's food library and the bolus schemes adjusted accordingly, as described above.

In yet another aspect of the invention, the delayed portion of the bolus split 404 can be delivered after a set period of time or, alternatively, the delayed portion of the bolus split 404 may be delivered at a constant, elevated rate. For example, five units can be delivered as an additional one unit per hour over five hours. This can be captured as a series of multi-wave deliveries, whose parameterization can be optimized based on a wide range of numerical capabilities. Specifically, the percentage for upfront delivery, the number of waves, the width of the waves, the duration of the waves and the total duration of each wave can all be parameterized and individualized on a per user basis.

$$U_{meal}(i) = U_{upfront}(i) + U_{wave}$$

$$U_{upfront}(i) = X \cdot U_{meal}(i)$$

$$U_{wave} = \sum_{i=1}^{n} U_n(i + j_n)$$

$$U_n(i + j_n) = \frac{X_n \cdot (1 - X) \cdot U_{meal}(i)}{d_n}$$

where:

$U_{meal}$ is a total amount of bolus for that meal;
$U_{upfront}$ is the total amount of insulin to be delivered at the time of the meal;
$U_{wave}$ is the total amount of insulin to be delivered as waves;
$X$ is the proportion of the total amount of insulin that will be delivered as waves;
$n$ is the number of bolus waves;
$j_n$ is the number of cycles after the initial upfront bolus where the subsequent bolus wave will be delivered;
$U_n(i + j_n)$ is the insulin delivery rate for the wave that will deliver over the $j_n$-th cycle;
$X_n$ is a proportion of the subsequent wave insulin that will be delivered in the $n$-th wave; and
$d_n$ is the duration of the $n$-th wave.

Each of the parameters of the equations above can be personalized based on the correlation between improved glucose control outcomes and the proportions of the contents of each recorded meal.

In a final aspect of the invention, bolus dosing application 156 may provide user feedback regarding any aspect of the delivery of the insulin. For example, user feedback may include meal catalogs and recommendations of favorable meals (i.e., past meals for which the post-prandial blood glucose metrics indicate that the blood glucose levels of the user were well-controlled, e.g., blood glucose readings between 70-180 mg/dL). In addition, the user may receive alerts, for example, mislabeled meals or unlabeled meals may be shown as alerts to the user with corrected labels and imputed labels based on the macronutrient profile prediction model 802.

Some examples of the disclosed system or methods may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:

1. A method for determining a bolus dose for a user comprising:

receiving a macronutrient profile for a meal;
determining an initial bolus dose;
predicting, based on the macronutrient profile, a post-prandial blood glucose trace comprising one or more blood glucose readings of the user at one or more time points in a post-prandial window, given an initial bolus dose;
iteratively evaluating the predicted blood glucose trace, adjusting the bolus dose, and re-predicting the blood glucose trace until the prediction shows desired blood glucose readings; and
providing an indication of the bolus dose that produced the prediction of the desired blood glucose trace.

2. The method of embodiment 1, wherein the bolus dose comprises a bolus quantity and a bolus split, the bolus split comprising a first portion of the bolus quantity to be administered at the start of the post-prandial window, and a second portion of the bolus quantity to be administered later in the post-prandial window.

3. The method of any of embodiments 1-2, wherein the predictions of the blood glucose trace are provided by a trained machine-learning model.

4. The method of any of embodiments 1-3, further comprising:

deriving a set of one or more metrics from the predicted blood glucose trace; and
using the derived metrics to evaluate the predicted blood glucose trace.

5. The method of any of embodiments 1-4, further comprising:

   receiving a blood glucose trace of the user from the post-prandial window; and
   updating the machine learning model using the received blood glucose trace or one or more metrics derived from the actual blood glucose trace all.

6. The method of any of embodiments 1-5, wherein the blood glucose trace of the user is received from a continuous glucose monitor worn by the user.

7. The method of any of embodiments 1-6, wherein the input to the machine-learning model comprises insulin on board for the user, a basal insulin rate and one or more current and recent blood glucose readings.

8. The method of any of embodiments 1-7, wherein the initial bolus dose is determined by:

   identifying, in a catalog of past meals consumed by the user, a closely-matched meal having a macronutrient profile that is a closest match to the macronutrient profile of the current meal;
   retrieving the bolus dose and the blood glucose trace for the closely-matched meal;
   adjusting the bolus dose to compensate for any undesirable blood glucose readings in the blood glucose trace for the closely-matched meal or for differences between the closely-matched meal and the current meal; and
   using the adjusted bolus dose as the initial bolus dose.

9. The method of any of embodiments 1-8, wherein the bolus dose that produced the prediction of the desired blood glucose trace is provided to an automatic drug delivery device that administers the first and second portions of the bolus dose to the user.

10. The method of any of embodiments 1-9, wherein the automatic drug delivery device receives information regarding the bolus dose that produced the prediction of the desired blood glucose trace via a wireless interface.

11. The method of any of embodiments 1-10, wherein the second portion of the bolus quantity is delivered at a predetermined time after the start of the post-prandial window.

12. The method of any of embodiments 1-11, wherein the second portion of the bolus quantity is delivered in one or more timed doses after the start of the post-prandial window.

13. The method of any of embodiments 1-12, wherein the timing of the delivery of the second portion of the bolus quantity is based on a characterization of the fat and protein concentrations in the macronutrient profile of the current meal.

14. The method of any of embodiments 1-13, wherein the machine-learning model comprises:

   a convolutional neural network that extracts one or more features from data input to the model; and
   a recurrent neural network that uses the features identified by the convolutional neural network to provide the prediction of the post-prandial blood glucose trace.

15. The method of any of embodiments 1-14, wherein the macronutrient profile of the meal is provided by the user.

16. The method of any of embodiments 1-15, wherein information regarding the meal is entered on an application running on a personal computing device of the user.

17. The method of any of embodiments 1-16, wherein the machine-learning model executes on a personal computing device of the user or is provided as a cloud-based service.

18. The method of any of embodiments 1-17, wherein the machine-learning model is initially trained on a wide population of users or a cluster of users similar to the user and further wherein the machine-learning model is updated based on subsequent meals entered by the user and the resulting post-prandial blood glucose traces.

19. A system comprising:

   a personal computing device of a user running an application enabling the user to input a macronutrient profile for a meal.
   a machine-learning model that predicts a post-prandial blood glucose trace given the macronutrient profile for the meal and an initial bolus dose; and
   an automatic drug delivery device in wireless communication with a personal computing device;
   wherein the initial bolus dose is determined based on the macronutrient profile of the meal and further wherein the initial bolus dose is iteratively adjusted until the machine-learning model predicts a post-prandial blood glucose trace having desired blood glucose readings.

20. The system of embodiment 19 further comprising:

   a continuous glucose monitor worn by the user and in wireless communication with the personal computing device;
   wherein the machine-learning model is updated using actual blood glucose readings from the continuous glucose monitor or using one or more metrics derived from the actual blood glucose readings.

**Claims**

1. A method for determining a bolus dose for a user comprising:

   receiving a macronutrient profile for a meal;
   determining an initial bolus dose;
   predicting, based on the macronutrient profile, a post-prandial blood glucose trace comprising one or more blood glucose readings of the user at one or more time points in a post-prandial window, given an initial bolus dose;
   iteratively evaluating the predicted blood glucose trace, adjusting the bolus dose, and re-predicting the blood glucose trace until the prediction shows desired blood glucose readings; and
   providing an indication of the bolus dose that produced the prediction of the desired blood glucose trace.

2. The method of claim 1 wherein the bolus dose comprises a bolus quantity and a bolus split, the bolus split comprising a first portion of the bolus quantity to be administered at the start of the post-prandial window, and a second portion of the bolus quantity to be administered later in the post-prandial window.

3. The method of claim 2, wherein the bolus dose that produced the prediction of the desired blood glucose trace is optionally provided to an automatic drug delivery device that administers the first and second portions of the bolus dose to the user, and wherein the automatic drug delivery device optionally receives information regarding the bolus dose that produced the prediction of the desired blood glucose trace via a wireless interface.

4. The method of claim 2 or 3 wherein the second portion of the bolus quantity is:

   delivered at a predetermined time after the start of the post-prandial window, or
   delivered in one or more timed doses after the start of the post-prandial window.

5. The method of any of claims 2-4 wherein the timing of the delivery of the second portion of the bolus quantity is based on a characterization of the fat and protein concentrations in the macronutrient profile of the current meal.

6. The method of any of claims 1-5 wherein the predictions of the blood glucose trace are provided by a trained machine-learning model, wherein the machine-learning model optionally executes on a personal computing device of the user or is provided as a cloud-based service.

7. The method of claim 6 further comprising:

   receiving a blood glucose trace of the user from the post-prandial window, wherein the blood glucose trace of the user is preferably received from a continuous glucose monitor worn by the user; and
   updating the machine learning model using the received blood glucose trace or one or more metrics derived from the actual blood glucose trace all.

8. The method of claim 6 or 7 wherein the input to the machine-learning model comprises insulin on board for the user, a basal insulin rate and one or more current and recent blood glucose readings.

9. The method of any of claims 6-8 wherein the machine-learning model comprises:

   a convolutional neural network that extracts one or more features from data input to the model; and
   a recurrent neural network that uses the features identified by the convolutional neural network to provide the prediction of the post-prandial blood glucose trace.

10. The method of any of claims 6-9 wherein the machine-learning model is initially trained on a wide population of users or a cluster of users similar to the user and further wherein the machine-learning model is updated based on subsequent meals entered by the user and the resulting post-prandial blood glucose traces.

11. The method of any of claims 1-10, further comprising:

    deriving a set of one or more metrics from the predicted blood glucose trace; and
    using the derived metrics to evaluate the predicted blood glucose trace.

12. The method of any of claims 1-11, wherein the initial bolus dose is determined by:

    identifying, in a catalog of past meals consumed by the user, a closely-matched meal having a macronutrient profile that is a closest match to the macronutrient profile of the current meal;
    retrieving the bolus dose and the blood glucose trace for the closely-matched meal;
    adjusting the bolus dose to compensate for any undesirable blood glucose readings in the blood glucose trace for the closely-matched meal or for differences between the closely-matched meal and the current meal; and

using the adjusted bolus dose as the initial bolus dose.

13. The method of any of claims 1-12 wherein the macronutrient profile of the meal is provided by the user, optionally entered on an application running on a personal computing device of the user.

14. A system comprising:

a personal computing device of a user running an application enabling the user to input a macronutrient profile for a meal.

a machine-learning model that predicts a post-prandial blood glucose trace given the macronutrient profile for the meal and an initial bolus dose; and

an automatic drug delivery device in wireless communication with a personal computing device;

wherein the initial bolus dose is determined based on the macronutrient profile of the meal and further wherein the initial bolus dose is iteratively adjusted until the machine-learning model predicts a post-prandial blood glucose trace having desired blood glucose readings.

15. The system of claim 14 further comprising:

a continuous glucose monitor worn by the user and in wireless communication with the personal computing device;

wherein the machine-learning model is updated using actual blood glucose readings from the continuous glucose monitor or using one or more metrics derived from the actual blood glucose readings.

Automatic Insulin Delivery (AID) System
100

**Personal Computing Device 150**
- Communication Interface 157
- GPS Receiver 159
- Processor 152
- User Interface 158
- Memory 154
  - Bolus Dosing Application 156

**Cloud-Based Services 160**

Wireless Commuinication Link 142

Wireless Communication Link 144

Wireless Communication Link 140

**Drug Delivery Device 105**
- Memory 104
  - Drug Delivery Application 106
- Communication Interface 114
- Power Source 112
- Processor 102
- Reservoir/ Pump 108
- Insulin Delivery Interface 110

**Sensor 180**
- Sensing / Measuring Device 182
- Communication Interface 184
- Power Source 186

Wireless Communication Link 146

FIG. 1

EP 4 086 914 A1

FIG. 2

Carbs | 15

Protein | 12

Fat | 15

**FIG. 3A**

C

F    P

**FIG. 3B**

1
1
1
1
2

Red Meat, Eggs

Fruits, Healthy Fats
nuts, seeds, era's, olive oil

Low GL Carbs
bread, lentils, whole grains

Protein
fruits, lean animal, low-fat dairy

Vegetables
mainly above-ground types

**FIG. 3C**

Bolus Insulin
**208**

Bolus Dose
**402**

Bolus Split
**404**

Post-Prandial Blood Glucose Prediction Model
**216**

Post-Prandial Blood Glucose Metrics
**406**

Recommended Bolus Dose & Split
**408**

*FIG. 4*

_500_

**FIG. 5**

510

512

Delayed
Hyperglycemia?
*602*

—Yes→

Increase Bolus Dose
by 10%
*604*

Early
Hyperglycemia?
*606*

—Yes→

Increase Split
Upfront Dose by 5%
*608*

No

Late
Hyperglycemia?
*610*

—Yes→

Decrease Split
Upfront Dose by 5%
*612*

506

## FIG. 6

502

504

Identify Previous
Closely Matched
Meals
**704**

Meal
Catalog
**702**

Obtain Post-
Prandial Metrics
from Previous
Closely Matched
Meals
**706**

Initial Bolus Insulin
**208**

Bolus Dose
**402**

Bolus Split
**404**

Adjust Bolus Dose/
Split from Previous,
Closely-Matched
Meals
**708**

506

*FIG. 7*

Post-Prandial Blood
Glucose Trace
**218**

Macronutrient
Profile Prediction
Model
**802**

Macronutrient Profile
**210**

Grams of
Carbohydrates

Grams of Fat

Grams of Protein

*FIG. 8*

# EP 4 086 914 A1

**EP 4 086 914 A1**

## EUROPEAN SEARCH REPORT

**Application Number**

EP 22 17 1736

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/125241 A1 (PRUD HOMME THIERRY [CH] ET AL) 20 May 2010 (2010-05-20) * The whole document, in particular: Paragraphs [0002], [0005], [0021] – [0031], [0052], [0056], [0058], [0071] – [0074], [0077] – [0081]; Figures 1, 4. * ----- | 1-15 | INV. G16H20/17 G16H50/70 A61M5/172 ADD. A61B5/145 |
| X | US 2020/268968 A1 (STEIL GARY [US] ET AL) 27 August 2020 (2020-08-27) * The whole document, in particular: Paragraphs [0016], [0017], [0037] – [0043], [0052], [0058] – [0062], [0065], [0111] – [0117], [0121], [0122], [0150], [0151] – [0153], [0165] – [0171], [0179] – [0181], [0189]; Figures 1, 2. * ----- | 1-15 | |
| X | US 2008/183060 A1 (STEIL GARRY M [US] ET AL) 31 July 2008 (2008-07-31) * The whole document, in particular: Paragraphs [0009] – [0011], [0048], [0049], [0089], [0090], [0126] – [0137]; Figures 1, 10, 11, 12A – 12D, 14. * ----- | 1-15 | |
| A | BELL KIRSTINE J. ET AL: "Optimized Mealtime Insulin Dosing for Fat and Protein in Type 1 Diabetes: Application of a Model-Based Approach to Derive Insulin Doses for Open-Loop Diabetes Management", DIABETES CARE, vol. 39, no. 9, 7 July 2016 (2016-07-07), pages 1631-1634, XP055961962, US ISSN: 0149-5992, DOI: 10.2337/dc15-2855 * The whole document, in particular: Abstract. * ----- -/-- | 1-15 | **TECHNICAL FIELDS SEARCHED (IPC)** G16H A61B A61M |

The present search report has been drawn up for all claims

| Place of search | Date of completion of the search | Examiner |
|---|---|---|
| Munich | 19 September 2022 | Nagele, Stefan |

Europäisches
Patentamt

European
Patent Office

Office européen
des brevets

**EUROPEAN SEARCH REPORT**

Application Number

EP 22 17 1736

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LI KEZHI ET AL: "Convolutional Recurrent Neural Networks for Glucose Prediction", IEEE JOURNAL OF BIOMEDICAL AND HEALTH INFORMATICS, IEEE, PISCATAWAY, NJ, USA, vol. 24, no. 2, 29 March 2019 (2019-03-29), pages 603-613, XP011770190, ISSN: 2168-2194, DOI: 10.1109/JBHI.2019.2908488 [retrieved on 2020-02-04] * The whole document, in particular: Abstract. * | 9 | |
| A | SAITI KYRIAKI ET AL: "A Review of Model Prediction in Diabetes and of Designing Glucose Regulators Based on Model Predictive Control for the Artificial Pancreas", 26 July 2017 (2017-07-26), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 66 - 81, XP047423422, ISBN: 978-3-540-74549-5 [retrieved on 2017-07-26] * The whole document, in particular: Abstract; Section 4.2. * | 9 | TECHNICAL FIELDS SEARCHED (IPC) |

The present search report has been drawn up for all claims

| Place of search | Date of completion of the search | Examiner |
|---|---|---|
| Munich | 19 September 2022 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 1736

This annex lists the patent family members relating to the patent documents cited in the above-mentioned European search report.
The members are as contained in the European Patent Office EDP file on
The European Patent Office is in no way liable for these particulars which are merely given for the purpose of information.

19-09-2022

| Patent document cited in search report | | Publication date | Patent family member(s) | | Publication date |
|---|---|---|---|---|---|
| US 2010125241 | A1 | 20-05-2010 | EP | 2350895 A2 | 03-08-2011 |
| | | | US | 2010125241 A1 | 20-05-2010 |
| | | | WO | 2010054777 A2 | 20-05-2010 |
| US 2020268968 | A1 | 27-08-2020 | US | 2020268968 A1 | 27-08-2020 |
| | | | WO | 2017184988 A1 | 26-10-2017 |
| US 2008183060 | A1 | 31-07-2008 | CA | 2672589 A1 | 07-08-2008 |
| | | | CA | 2919921 A1 | 07-08-2008 |
| | | | DK | 2114239 T3 | 07-07-2014 |
| | | | EP | 2114239 A1 | 11-11-2009 |
| | | | EP | 2425772 A1 | 07-03-2012 |
| | | | EP | 2425773 A1 | 07-03-2012 |
| | | | EP | 2425774 A1 | 07-03-2012 |
| | | | JP | 5538904 B2 | 02-07-2014 |
| | | | JP | 2010523167 A | 15-07-2010 |
| | | | JP | 2014204998 A | 30-10-2014 |
| | | | US | 2008183060 A1 | 31-07-2008 |
| | | | US | 2011282320 A1 | 17-11-2011 |
| | | | US | 2011282321 A1 | 17-11-2011 |
| | | | US | 2019053742 A1 | 21-02-2019 |
| | | | US | 2021038134 A1 | 11-02-2021 |
| | | | WO | 2008094249 A1 | 07-08-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63184241